# EUROPÄISCHE PATENTANMELDUNG

(11) **EP 0 791 830 A1**
(43) Veröffentlichungstag der Anmeldung: **27.08.1997**
(21) Anmeldenummer: 96102716.6
(22) Anmeldetag: 23.02.1996
(51) Int. Cl.: G01N 33/543, G01N 33/547

(54) **Verfahren zur Kopplung von Substanzen an Festphasen und Trägersystem für den Nachweis von Allergien**

(71) Anmelder: Schwertner, Heiko, Dr. rer. nat., 35039 Marburg (DE)
(72) Erfinder: Schwertner, Heiko, Dr. rer. nat., 35039 Marburg (DE)
(74) Vertreter: Olbricht, Karl Heinrich, Dipl.-Phys.

(57) **Zusammenfassung**

Ein Verfahren zur Kopplung von Substanzen, insbesondere von Allergenen, an Festphasen verwendet erfindungsgemäß heterobifunktionelle Verbindungen wie z.B. Azidobenzoesäurederivate mit wenigstens zwei unterschiedlichen funktionellen Gruppen pro Molekül, wobei eine erste Gruppe das heterobifunktionelle Molekül kovalent an die Festphase bindet und eine zweite Gruppe, z.B. eine Arylazid-Gruppe, chemisch aktivierbar, insbesondere photoaktivierbar ist. An dem heterobifunktionellen Molekül, das auch über ein Protein oder ein Proteingemisch an die Festphase gebunden sein kann, setzt zur Steuerung der erforderlichen Aktivierungsenergie der Arylazid-Gruppe bevorzugt eine dritte Gruppe an, beispielsweise eine Nitro-Gruppe. Das zu koppelnde Allergen wird in eine bevorzugt saure Lösung gebracht, die stark verdünnte HCl-Lösung und ein organisches Lösemittel, beispielsweise Dioxan, enthalten kann.

## Beschreibung

Die Erfindung betrifft ein Verfahren zur Kopplung von Substanzen an eine Festphase gemäß dem Oberbegriff von Anspruch 1, die Verwendung von heterobifunktionellen Verbindungen gemäß Anspruch 15 sowie ein Trägersystem für den Nachweis von Allergien mittel*s in vitro* Allergiediagnostikverfahren gemäß dem Oberbegriff von Anspruch 16.

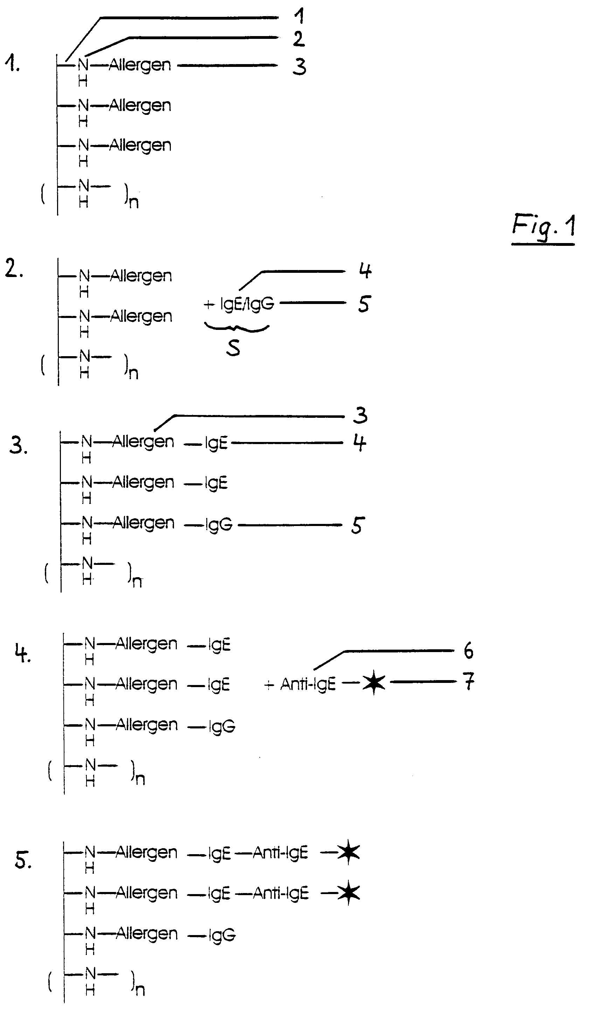

In der *in vitro* Allergiediagnostik werden mittels Reaktionen, die durch den Kontakt von Allergenen mit Antikörpern, Immunkomplexen oder sensibilisierten Immunzellen des Probanden auftreten, Allergien nachgewiesen. So wird beispielsweise in einem in Fig. 1 schematisch dargestellten Verfahren eine vermutlich allergen wirkende Substanz 3 über ihre freien Amino- oder Imino-Gruppen 2 an eine Festphase 1 oder ein Fasernetzwerk gekoppelt (Fig. 1.1) und mit einem zu untersuchenden Serum S in Kontakt gebracht (Fig. 1.2). Sind darin Antikörper 4, 5 entsprechender Spezifität vorhanden, werden diese von den Testallergenen 3 gebunden (Fig. 1.3). Nach einer ausreichenden Inkubationszeit wird der Träger 1 zum Entfernen nicht gebundener Antikörper mit einer Pufferlösung gründlich gewaschen und getrocknet. Anschließend wird eine Lösung mit markierten Anti-IgE (Reaginen 6) zugesetzt und erneut inkubiert (Fig. 1.4). Kommt es zu einer Bindung der Reagine 6 an das Allergosorbens (Fig. 1.5), ist die Häufigkeit dieser Bindungen proportional zur Anzahl der aus dem zu untersuchenden Patientenserum gebundenen IgE. Je nach Art der Markierung 7 werden verschiedene Verfahren unterschieden (RAST, EAST, RIST, ELISA).

Für die Durchführung der genannten Diagnostikverfahren werden als Festphasen bzw. Fasernetzwerke Trägerscheiben oder -plättchen aus Cellulose, Nylon o.dgl. verwendet, die mit den vermutlich allergen wirkenden Substanzen präpariert sind und in Mikrotiterplatten oder Röhrchen als Reaktionsgefäße automatisiert abgearbeitet werden können. Anwendung als Träger finden auch Gele aus Agarose, Sepharose o.dgl. oder mit Allergenen beschichtete Mikrotiterplatten aus Polystyrol.

Die Kopplung der für eine Versuchsreihe ausgewählten Allergene an Cellulose- oder Nylon-Trägern erfolgt herkömmlich über eine spezifische funktionelle Gruppe, beispielsweise eine CNBr-Gruppe. Dabei kann jedoch das Allergen nur über die freien Amino- oder Imino-Gruppen gebunden werden. Moleküle oder Substanzen, die keine NH₂- oder NH-Gruppen aufweisen, sind nicht einsetzbar und können daher nicht identifiziert werden. Ein weiterer Nachteil der CNBr-Kopplung besteht in der extremen Umweltbelastung. Bei der Aktivierung des Trägers entsteht Bromwasserstoffsäure (HBr) und Blausäure (HCN), die beide hochgiftig sind. Die Arbeit im Labor muß unter erhöhten Sicherheitsbedingungen erfolgen; die Entsorgung der Abfall- und Reststoffe ist entsprechend aufwendig und teuer.

Bei der Verwendung von Trägern mit freien OH-Gruppen müssen diese zu Carboxyl-Gruppen umgewandelt werden. Das Allergen kann anschließend über eine Aktivierung mit Dicyclohexylcarbodiimid (DCC) wiederum nur über NH₂-Gruppen gebunden werden, so daß ebenfalls nur ein begrenzter Anwendungsbereich gegeben ist.

Für jede Substanz oder Substanzklasse ist eine individuell angepaßte Kopplungs-Methodik erforderlich, die für jede neu auftretende, spezielle Fragestellung entwickelt werden muß. Dies hat zur Folge, daß viele Substanzen einer allergischen Identifizierung entgehen, da mit den bekannten *in vitro* Verfahren nur die häufiger auftretenden und dementsprechend in der Literatur beschriebenen Allergene standardmäßig bestimmt werden können. Kurzfristige Untersuchungen im Labor mit unbekannten Substanzen sind daher nur mit erheblichem Aufwand möglich. Die bei der Entwicklung eines spezifischen Kopplungsverfahrens entstehenden Kosten sind oft im Einzelfall daher nicht vertretbar, so daß eine rasche und gezielte Therapie des Patienten verhindert wird. Wenn Substanzen oder Substanzgemenge, die bei einem einzelnen Patienten eine allergische Reaktion auslösen, nicht bestimmt werden können, besteht keine Möglichkeit, eine *in vitro* Allergiediagnose durchzuführen, d.h. unter Verwendung von allergenem Material, das nur individuell bei einem einzelnen Patienten eine Allergie ausgelöst hat. Eine gezielte Behandlung eines solchen Patienten ist nicht möglich.

Ziel der Erfindung ist es, im diagnostischen Routinebetrieb eine schnelle und einfache Kopplung beliebiger Substanzen oder Substanzgemische an eine Festphase zu ermöglichen. Ein entsprechendes Verfahren soll einfach und kostengünstig anwendbar sein. Ferner sollen Umweltbelastungen nahezu vollständig vermieden werden. Ein weiteres Ziel der Erfindung besteht darin, einen mit beliebigen, insbesondere in ihrem Verhalten unbekannten, Allergenen gekoppelten Träger oder Tragkörper zu entwickeln, der unmittelbar in bekannten *in vitro* Allergiediagnostik-Verfahren eingesetzt werden kann.

Hauptmerkmale der Erfindung sind in Anspruch 1, 14 und 15 angegeben. Ausgestaltungen sind Gegenstand der Ansprüche 2 bis 13 und 16 bis 20.

Zur Lösung der Aufgabe wird bei einem Verfahren zur Kopplung von Substanzen, insbesondere von Allergenen, die Verwendung einer heterobifunktionellen Verbindung mit wenigstens zwei unterschiedlichen funktionellen Gruppen pro Molekül vorgeschlagen, wobei eine erste Gruppe das heterobifunktionelle Molekül kovalent an die Festphase bindet und eine zweite Gruppe chemisch aktivierbar ist. Die Kopplung eines Allergens ist somit nicht mehr auf die Gegenwart einer freien spezifischen Gruppe angewiesen. Sie erfolgt vielmehr unter Zwischenschaltung eines Moleküls, das mit einer chemisch aktivierbaren Gruppe die verschiedensten Allergendeterminanten oder -orientierungen mittels einer unspezifisch kovalenten Bindung an die Festphase binden kann. Darüber hinaus lassen sich mit Hilfe des erfindungsgemäßen Verfahrens auch andere beliebige Substanzen oder Komponenten an eine Festphase koppeln, die damit auch in anderen Analyse- oder Diagnoseverfahren einsetzbar sind.

Gemäß Anspruch 2 wird das heterobifunktionelle Molekül mit der ersten Gruppe über eine funktionelle Gruppe an die Festphase gebunden. Dies kann beispielsweise über ein Protein erfolgen, welches selbst kovalent an die Festphase gebunden ist. Dadurch wird für die zu koppelnden Allergene eine körperverwandte Umgebung geschaffen, was zu aussagekräftigeren analytischen Ergebnissen führt. Die Anwendung des Verfahrens ist nicht mehr auf die Verwendung bestimmter Festphasen beschränkt. Vielmehr können beliebige Trägermaterialien und -zusammensetzungen individuell an die Bindungseigenschaften des heterobifunktionellen Moleküls und/oder der Allergene angepaßt werden. Hierfür eignen sich z.B. Carboxyl-Gruppen, die sich mittels DCC einfach an NH-, NH₂- oder OH-Gruppen binden lassen. Anspruch 3 sieht anstelle eines Proteins ein Proteingemisch vor, das laut Anspruch 4 humanes Gewebe enthalten kann. Die Anwendungsmöglichkeiten werden somit zusätzlich erweitert.

Die zweite Gruppe des heterobifunktionellen Moleküls ist nach Anspruch 5 photoaktivierbar, was eine Aufteilung des Verfahrens in zwei Phasen ermöglicht. Die photoaktive Gruppe ist während der Kopplung des Moleküls an die Festphase inaktiv, so daß keine unerwünschten Reaktionen stattfinden und eine Zwischenlagerung der vorpräparierten Festphasen problemlos möglich ist. Erst durch Aktivierung der photoaktiven Gruppe, beispielsweise durch Bestrahlung, erfolgt eine Ankopplung der zu analysierenden Substanzen an die Festphase, wobei die dabei ablaufenden, photochemisch induzierten Bindungsreaktionen gezielt gesteuert werden können. Nach der Aktivierung ist die Reaktion in wenigen Minuten abgeschlossen. Um die Lagerungseigenschaften und -möglichkeiten weiter zu verbessern, ist die zweite Gruppe laut Anspruch 6 in wäßriger oder organischer Lösung stabil.

Von besonderer Bedeutung ist die in Anspruch 7 angegebene Gestaltung des erfindungsgemäßen Verfahrens, wonach die zweite Gruppe durch Aktivierung einen radikalischen Reaktionsmechanismus durchläuft. Die heterobifunktionelle Verbindung ist damit in der Lage, beliebige unspezifisch funktionelle Gruppen wie NH₂, NH, OH, SH, COOH, schwach aktivierte Doppelbindungen, elektronegative oder elektropositive Bereiche einer Substanz zu binden. Es können viele Haptendeterminanten oder -orientierungen kovalent an die Festphase gebunden werden. Die Zahl der möglichen Bindungsstellen für potentielle Antikörper ist nahezu unbeschränkt, so daß langwierige und aufwendige Versuchsreihen für die Ankopplung selten auftretender Allergene nicht mehr erforderlich sind. Diese lassen sich vielmehr unmittelbar nach ihrer Gewinnung in einem unkompliziert zu handhabenden Prozeß an eine gewünschte Festphase koppeln und unmittelbar einem geeigneten Analyseverfahren zuführen. Eine gezielte Behandlung des Patienten kann - sofern notwendig - sofort eingeleitet werden.

Anspruch 8 sieht in der zweiten Gruppe des heterobifunktionellen Moleküls bevorzugt die Verwendung einer Arylazid-Gruppe vor. Diese gestattet problemlos die Zwischenlagerung der aktivierten Festphase, da die Arylazid-Gruppe in dunklem, trockenem und kühlem Zustand über längere Zeit stabil bleibt. Durch die Bestrahlung mit UV-Licht verschiedener Wellenlängen wird die Arylazid-Gruppe photochemisch zu einem Radikal aktiviert, das eine Vielzahl von Molekülen unspezifisch bindet. Die Zahl der detektierbaren Antikörper ist nicht mehr auf einige wenige gegebenenfalls in der Literatur beschriebene Allergene beschränkt.

In der Ausbildung von Anspruch 9 setzt an dem heterobifunktionellen Molekül eine weitere Gruppe an, beispielsweise eine Nitro-Gruppe. Mit dieser kann die zur Aktivierung der photoaktiven Gruppe notwendige Energie variiert werden. Durch die Anlagerung einer Nitro-Gruppe verlängert sich beispielsweise die für die Aktivierung erforderliche Wellenlänge, so daß preisgünstigere UV-Lampen eingesetzt werden können.

Bevorzugt ist das heterobifunktionelle Molekül laut Anspruch 10 ein Azidobenzoesäurederivat, das in beliebigen Lösungsmitteln verwendbar und als günstiges Reagenz leicht und kostengünstig zu beschaffen ist. Als Festphase sieht Anspruch 11 die Verwendung einer Matrix aus Cellulose, Nylon, Agarose, Sephadex o.dgl. vor, die in beliebiger Trägerform als Scheiben, Plättchen oder Gel verfügbar sind. Die mit diesen Materialien bereits gewonnenen Erkenntnisse lassen sich damit problemlos übernehmen. Ihre Beschaffung und Lagerung ist leicht und kostengünstig.

Eine wichtige Weiterbildung geht aus Anspruch 12 hervor. Danach wird die an die Festphasenträger zu koppelnde Substanz in Lösung gebracht, die bevorzugt sauer ist. Die Zuführung der zu untersuchenden Substanzen an die photoaktive Festphase erfolgt somit besonders einfach mittels einer Lösung, die dem pH-Wert des menschlichen Körpers angepaßt ist. Zudem lassen sich selbst unterschiedliche Feststoffe oder Materialien mit der Festphase in Kontakt bringen, was die Anwendungsvielfalt der präparierten Festphasen drastisch erhöht. Ferner ist es durch den gezielten Einsatz von Lösemitteln oder -gemischen möglich, aus einem Substanzgemisch ganz bestimmte Fraktionen von Substanzen für die Kopplung zu separieren. Vorzugsweise enthält die Lösung gemäß Anspruch 13 stark verdünnte HCl-Lösung und ein organisches Lösemittel, beispielsweise Dioxan. Diese Substanzen gehören zur Standardausrüstung eines Labors und stehen dadurch bequem zur Verfügung. Ferner können diese Lösungen direkt für Epikutan-Tests an Patienten eingesetzt werden.

Anspruch 14 sieht die Verwendung von heterobifunktionellen Verbindungen mit wenigstens zwei unterschiedlichen funktionellen Gruppen pro Molekül zur Kopplung von Substanzen, insbesondere Allergenen, an Festphasen vor, wobei eine erste Gruppe das heterobifunktionelle Molekül kovalent an die Festphase bindet und eine zweite Gruppe photochemisch aktivierbar ist.

Wichtige Vorteile der Erfindung bestehen darin, daß ein diagnostischer Routinebetrieb mit neuen, bisher noch nicht standardisierten Allergenen rasch und kostengünstig aufgebaut werden kann. Reihenuntersuchungen ganzer Bevölkerungskollektiven, die mit einer allergenen Substanz in Kontakt gekommen sind, lassen sich problemlos und kostengünstig durchführen. Aufgrund der vielfältigen Bindungsmöglichkeiten des heterobifunktionellen Moleküls sind keine komplizierten chemischen Kopplungsmethoden und damit auch keine Spezialkenntnisse bei der Durchführung der Kopplung mehr notwendig. Ein Arzt oder eine Laborantin kann unmittelbar nach Gewinnung einer Probe die erforderlichen Schritte zur Kopplung des Allergens an die Festphase selbständig durchführen. Ferner entstehen während der Kopplung keinerlei giftige oder umweltbelastende Dämpfe. Alle löslichen Produkte lassen sich einfach und umweltfreundlich entsorgen.

Bei einem Trägersystem für den Nachweis von Allergien mittels *in vitro* Allergiediagnostikverfahren bestehend aus Trägerelementen und an deren Oberfläche gekoppelten Allergenen sind letztere nach Anspruch 15 über heterobifunktionelle Moleküle kovalent an freie Bindungsstellen der Trägeroberfläche gebunden. Die Trägerelemente des Trägersystems können somit die unterschiedlichsten Allergene tragen, insbesondere auch solche, die keine spezifischen Bindungsgruppen aufweisen. Vielmehr lassen sich über die heterobifunktionellen Moleküle auch bisher unbekannte, noch nicht standardisierte allergische Substanzen an die Trägerelemente koppeln und unmittelbar in einem *vitro* Allergiediagnostikverfahren einsetzen. Die langwierige Entwicklung von individuell angepaßten Kopplungsmechanismen entfällt.

Gemäß Anspruch 16 weisen die heterobifunktionellen Moleküle wenigstens zwei funktionelle Gruppen auf, so daß mit einer ersten Gruppen das Molekül an das Trägerelement und mit einer zweiten Gruppe das Allergen an das Molekül gebunden wird. Dabei ist es von Vorteil, wenn nach Anspruch 17 eine der funktionellen Gruppen zur Bindung der Allergene photoaktivierbar ist. Das Trägersystem läßt sich individuell konfektioniert und kostengünstig herstellen. Anspruch 18 sieht ferner vor, daß das heterobifunktionelle Molekül ein Azidobenzoesäurederivat ist.

Um die Bindungsmöglichkeiten für die Allergene weiter zu verbessern sind gemäß Anspruch 19 zwischen dem heterobifunktionellen Molekül und der Trägeroberfläche des Trägers Proteine oder Proteingemische angeordnet. Diese simulieren körpereigene Bedingungen, was die Reaktionsausbeute der Allergene positiv beeinflußt.

Für den Einsatz der Trägersysteme im automatisierten Routinebetrieb ist es von Vorteil, daß laut Anspruch 20 die Trägerelemente als Matrix in Form von Scheiben, Plättchen, Gel oder ähnlichen Substraten ausgebildet sind, wobei die Scheiben oder Plättchen nach Anspruch 21 aus Cellulose, Nylon o.dgl. bestehen und das Gel gemäß Anspruch 22 aus Agarose, Sephadex o.dgl. besteht.

Vorteilhaft ist die Maßnahme von Anspruch 23, wonach die Trägerelemente in Reaktionsgefäße eingesetzt sind. Die Allergenträger können unmittelbar im Labor abgearbeitet werden. Die Reaktionsgefäße dienen gleichzeitig als Transportgefäße.

Weitere Merkmale, Einzelheiten und Vorteile der Erfindung ergeben sich aus dem Wortlaut der Ansprüche sowie aus der folgenden spezielleren Beschreibung. Es zeigen:
- Fig. 1: ein herkömmliches *in vitro* Allergiediagnostik-Verfahren,
- Fig. 2: wichtige Verfahrensstufen des erfindungsgemäßen Verfahren und
- Fig. 3: ein anderes Ausführungsbeispiel des Verfahrens von Fig. 2.

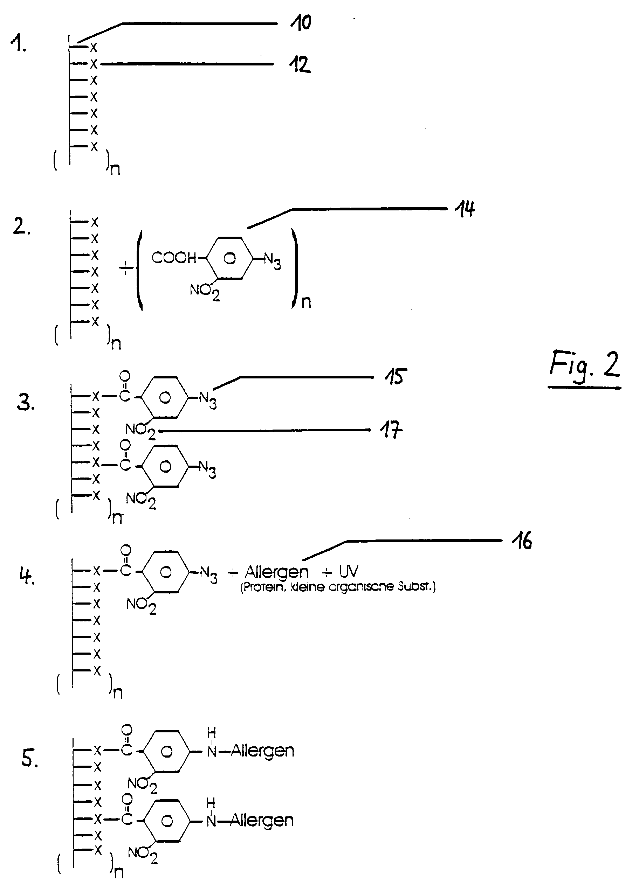

Das in Fig. 2 dargestellte Kopplungsverfahren ist im wesentlichen in zwei Phasen unterteilt. In der ersten Phase (Fig. 2.1 bis 2.3) erfolgt eine Aktivierung eines Trägermaterials, in der zweiten Phase (Fig. 2.4 und 2.5) wird das zu untersuchende allergene Material an den Festphasen-Träger gekoppelt.

Zunächst werden mit Human Serum Albumin (HSA) belegte Cellulose-Scheiben 10 dreimal mit H₂O bi, einmal mit EtHO, einmal mit Aceton und einmal mit Hexan gewaschen. Die einzeln auf Edelstahlstecknadeln gesteckten Scheiben werden an der Luft getrocknet. Die auf den Scheiben 10 zur Verfügung stehenden freien Bindungsstellen sind in Fig. 2 allgemein mit 12 bezeichnet.

Für die Kopplung der Trägerscheiben mit dem Photoreagenz (Fig. 2.2) werden in 50 ml Dioxan unter Lichtabschluß 100 mg 5-Azido-2-nitrobenzoesäure (0,7 mmol) mit 140 mg Dicyclohexylcarbodiimid (0,72 mmol) bei Raumtemperatur für etwa 2 min gelöst. Anschließend werden die gereinigten, mit HSA belegten Scheiben in die Lösung getaucht und für zwei Stunden bei Raumtemperatur darin leicht bewegt. Dabei geht das Azidobenzoesäurederivat 14 als heterobifunktionelles Molekül mit seiner Carboxyl-Gruppe eine Estherbindung mit den Bindungsstellen 12 des Cellulose-Trägers 10 ein (Fig. 2.3). Um eine hohe Reaktionsausbeute zu erzielen, müssen die Lösungsmittel so weit wie möglich wasserfrei sein. Hierfür empfiehlt sich die Behandlung des Dioxan mit trockenem MgSO₄ kurz vor der Reaktion. Im Anschluß daran wird die Reaktionslösung abgegossen und die Scheiben zweimal mit 50 ml Dioxan gewaschen. Die Trocknung erfolgt ebenfalls unter Lichtabschluß.

Die Vorbereitung der zu analysierenden Allergenlösungen hängt von der Beschaffenheit des Ausgangsmaterials ab. Allergenmaterial, das in Pulver- oder Tablettenform vorliegt, wird zu 100 mg in 250 µl 0.1M-NaHCO₃ oder 0.1M-HCl suspendiert und bei Raumtemperatur für etwa 2 min vermischt. Nach Zugabe von 250 µl Dioxan wird erneut für etwa 2 min gerührt.

Organische Substanzgemenge wie Fette, Öle, Lacke, Salben o.dgl. werden zu 100 mg unter Zugabe von 500 µl Toluol, Hexan oder Dichlorethan (1/1/1; V/V/V) bei Raumtemperatur gut vermischt, bis sich eine wäßrige Lösung von der organischen Lösung absetzt. Die wäßrige Lösung wird abgeschöpft und weiterverwendet.

Nicht lösbare Partikel werden nach Möglichkeit zu Staub zerkleinert. 100 mg davon werden mit 250 µl Dioxan/0.1 M NaHCO₃ (1/1; V/V) versetzt und 30 min bei Raumtemperatur zu einer Suspension vermischt. Diese kann direkt verwendet werden.

Extrakte aus Feststoffen wie Teppichen, Tapeten, Federn, Matratzen, Haaren o.dgl. werden feinst zerkleinert und mit dem 10fachen Volumen H₂O bi über Nacht unter Schütteln bei Raumtemperatur angesetzt. Anschließend wird die Lösung für 10 min mit 5000 U/min zentrifugiert. Die zerkleinerten Feststoffe werden abgefangen und diesmal mit dem 5fachem Volumen Hexan/Dichlorethan (1/1; V/V) über Nacht unter Schütteln bei Raumtemperatur angesetzt. Die organische Phase wird soweit eingeengt, bis in der Lösung gerade nichts mehr ausfällt. Danach wird die wäßrige Phase gefriergetrocknet und die Restsubstanz in H₂O bi so konzentriert wie möglich aufgenommen. Sowohl die organische Phase als auch die wäßrige Suspension können für die Belegung der Scheiben eingesetzt werden.

In der zweiten Phase des Verfahrens erfolgt die Kopplung der Photoreagenz-Scheiben 10 mit dem zu überprüfenden Testallergen 16. Dazu werden 500 µl einer zuvor angesetzten Allergenlösung in einen Eppendorfcup gefüllt. Anschließend wird eine der präparierten Scheiben unter Lichtabschluß darin eingetaucht, nach einer kurzen Einwirkzeit ans Tageslicht geholt und schließlich in 100 µl Allergenlösung bei Raumtemperatur für 15 min mit einer UV-Lampe bestrahlt (Fig. 2.4). Das Wellenlängenspektrum der Lampe liegt im Bereich zwischen 260 bis 280 nm und ist geeignet, die Arylazid-Gruppe 15 zu aktivieren. Zum Abschluß wird die Lösung mit der Scheibe für etwa 30 min dem Tageslicht ausgesetzt oder mit einer Halogenlampe bestrahlt. Um die für die Aktivierung der Arylazid-Säure erforderlichen Wellenlängen in den Bereich des sichtbaren Lichts zu verschieben, ist an dem Benzolring des Azidobenzoesäurederivats 14 eine Nitro-Gruppe 17 angelagert, was die Verwendung von handelsüblichen, preiswerten UV-Strahlern ermöglicht.

Fig. 2.5 zeigt eine fertig präparierte Trägerscheibe 10, die an ihren freien Bindungsstellen 12 über eine Esterverbindung das heterobifunktionelle Molekül 14 und über eine Imino-Gruppe das zu testende Allergen trägt. Die Arylazid-Gruppe 15 des Azidobenzoesäurederivats 14 ist allerdings auch in der Lage beliebige spezifisch und/oder unspezifisch funktionelle Gruppen wie NH₂, OH, SH, COOH, schwach aktivierte Doppelbindungen, elektronegative oder elektropositive Bereiche einer Substanz zu binden, so daß eine Vielfalt von in ihrem Verhalten bekannten und unbekannten Allergenen bzw. allergen wirkenden Substanzen an die Trägerscheibe gebunden werden können.

Die mit der Azidobenzoesäure belegten Cellulose-Träger können entweder in der Allergenlösung oder in trockenem Zustand unter Lichtabschluß für etwa zwei Tage gelagert werden. Wäscht man die Scheiben mit einer 0.1 M NaCO₃-Lösung ist nach der Trocknung eine Aufbewahrung für etwa zwei Monate möglich. In feuchtem Zustand halten sich die Scheiben bis zu zwei Wochen.

Für die Herstellung von Referenzscheiben werden die entsprechend der Phase 1 präparierten Scheiben unter Lichtabschluß in einen mit 500 µl H₂O bi befüllten Eppendorfcup gelegt, anschließend ans Tageslicht geholt und in 100 µl H₂O bi bei Raumtemperatur für 15 min mit UV-Licht bestrahlt. Die Wellenlänge liegt bei 260 bis 280 nm. Schließlich wird das Wasser mit den Scheiben für etwa 30 min dem Tageslicht ausgesetzt oder mit einer Halogenlampe bestrahlt. In dem Wasser halten sich die Cellulose-Träger für etwa zwei Tage. Für eine Verlängerung der Aufbewahrungszeit müssen sie mit einer 0.1 M NaCO₃-Lösung gewaschen und getrocknet werden.

Die fertig präparierten Cellulose-Scheiben 10 bilden somit als Trägerelemente ein Trägersystem für den Nachweis von Allergien und werden für den Einsatz in einem *in vitro* Allergietest in Reaktionsgefäße eingesetzt werden. Diese können in Form einer Matrix in einem verschließbaren Containern angeordnet werden, der sowohl als Arbeits- als auch als Transportgefäß verwendet werden kann. Die Test-Allergene 16 sind über ein Azidobenzoesäurederivat und dessen funktionelle Gruppen kovalent an freie Bindungsstellen 12 der Scheibenoberfläche gebunden, wobei zumindest eine Gruppe photoaktivierbar ist.

Die Trägerelemente des Trägersystems können auch als Matrix in Form von Scheiben, Plättchen, Gel oder ähnlichen Substraten ausgebildet, wobei die Scheiben oder Plättchen bevorzugt aus Cellulose, Nylon o.dgl. bestehen, während als Gel Agarose, Sephadex o.dgl. verwendet wird.

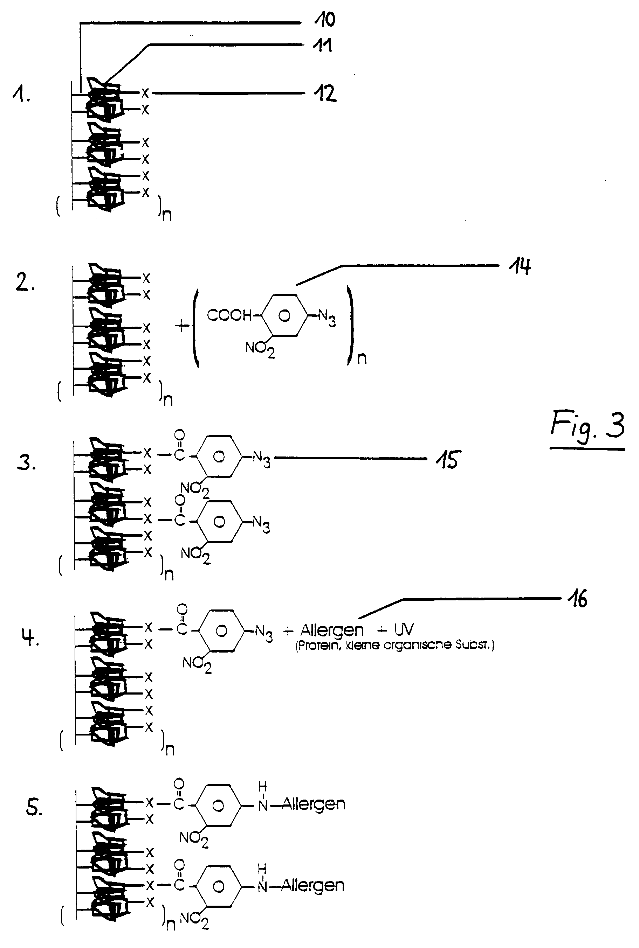

In einem anderen Ausführungsbeispiel des erfindungsgemäßen Verfahrens, dargestellt in Fig. 3, erfolgt die Bindung des heterobifunktionellen Photoreagenz 14 an die Trägerelemente 10 über Proteine 11 oder Proteingemische, beispielsweise aus humanem Gewebe. Diese simulieren körperähnliche Umgebungsverhältnisse für die Allergene, so daß diese leichter über die heterobifunktionellen Moleküle gebunden werden. Man erzielt besonders aussagekräftige Analyseergebnisse.

Die Erfindung ist nicht auf eine der vorbeschriebenen Ausführungsformen beschränkt, sondern in vielfältiger Weise abwandelbar. So lassen sich mit diesem Verfahren Substanzen oder Substanzgemische an Festphasen koppeln, die bequem auch in anderen Verfahren eingesetzt werden können, beispielsweise in der Analyse von Autoimmunantikörpern, in der Analyse von DNA/RNA oder in der Proteinreinigung.

Man erkennt, daß ein Verfahren zur Kopplung von Substanzen, insbesondere von Allergenen, an Festphasen erfindungsgemäß heterobifunktionelle Verbindungen wie z.B. Azidobenzoesäurederivate mit wenigstens zwei unterschiedlichen funktionellen Gruppen pro Molekül verwendet, wobei eine erste Gruppe das heterobifunktionelle Molekül kovalent an die Festphase bindet und eine zweite Gruppe, z.B. eine Arylazid-Gruppe, chemisch aktivierbar, insbesondere photoaktivierbar ist. An dem heterobifunktionellen Molekül, das auch über ein Protein oder ein Proteingemisch an die Festphase gebunden sein kann, setzt zur Steuerung der erforderlichen Aktivierungsenergie der Arylazid-Gruppe bevorzugt eine dritte Gruppe an, beispielsweise eine Nitro-Gruppe. Das zu koppeinde Allergen wird in eine bevorzugt saure Lösung gebracht, die stark verdünnte HCl-Lösung und ein organisches Lösemittel, beispielsweise Dioxan, enthalten kann.

Sämtliche aus den Ansprüchen, der Beschreibung und der Zeichnung hervorgehenden Merkmale und Vorteile, einschließlich technischer Einzelheiten und Verfahrensschritte, können sowohl für sich als auch in den verschiedensten Kombinationen erfindungswesentlich sein.

### Bezugszeichenliste

- S: Serum
- 1: Festphase
- 2: freie Imino-Gruppe
- 3: Testallergen
- 4, 5: Antikörper
- 6: Reagin
- 7: Markierung
- 10: Träger
- 11: Protein
- 12: freie Bindungsstelle
- 14: heterobifunktionelles Molekül
- 15: Arylazid-Gruppe
- 16: Testallergen
- 17: Nitrid-Gruppe

## Patentansprüche

1. Verfahren zur Kopplung von Substanzen, insbesondere von allergen wirkenden Substanzen oder Allergenen, an Festphasen, **gekennzeichnet** durch die Verwendung einer heterobifunktionellen Verbindung mit wenigstens zwei unterschiedlichen funktionellen Gruppen pro Molekül, wobei eine erste Gruppe das heterobifunktionelle Molekül kovalent an die Festphase bindet und eine zweite Gruppe chemisch aktivierbar ist.

2. Verfahren nach Anspruch 1, dadurch **gekennzeichnet**, daß das heterobifunktionelle Molekül mit der ersten Gruppe über eine funktionelle Gruppe, beispielsweise ein Protein, an die Festphase gebunden wird.

3. Verfahren nach Anspruch 1 oder 2, dadurch **gekennzeichnet,** daß das heterobifunktionelle Molekül mit der ersten Gruppe über ein Proteingemisch an die Festphase gebunden wird.

4. Verfahren nach Anspruch 3, dadurch **gekennzeichnet**, daß das Proteingemisch humanes Gewebe enthält.

5. Verfahren nach einem der Ansprüche 1 bis 4, dadurch **gekennzeichnet**, daß die zweite Gruppe photoaktivierbar ist.

6. Verfahren nach Anspruch 5, dadurch **gekennzeichnet**, daß die zweite Gruppe in wäßriger oder organischer Lösung stabil ist.

7. Verfahren nach Anspruch 5 oder 6, dadurch **gekennzeichnet**, daß die zweite Gruppe durch Aktivierung einen radikalischen Reaktionsmechanismus durchläuft (radikal reagiert).

8. Verfahren nach einem der Ansprüche 5 bis 7, dadurch **gekennzeichnet**, daß die zweite Gruppe bevorzugt eine Arylazid-Gruppe ist.

9. Verfahren nach einem der Ansprüche 1 bis 8, dadurch **gekennzeichnet**, daß an dem heterobifunktionellen Molekül eine dritte Gruppe, beispielsweise eine Nitro-Gruppe, ansetzt.

10. Verfahren nach einem der Ansprüche 1 bis 9, dadurch **gekennzeichnet**, daß das heterobifunktionelle Molekül ein Azidobenzoesäurederivat ist.

11. Verfahren nach einem der Ansprüche 1 bis 10, dadurch **gekennzeichnet**, daß als Festphase eine Matrix aus Cellulose, Nylon, Agarose, Sephadex o.dgl. verwendet wird.

12. Verfahren nach einem der Ansprüche 1 bis 11, dadurch **gekennzeichnet**, daß die zu koppelnde Substanz in eine Lösung gebracht wird, die bevorzugt sauer ist.

13. Verfahren nach Anspruch 12, dadurch **gekennzeichnet**, daß die Lösung stark verdünnte HCl-Lösung und ein organisches Lösemittel, beispielsweise Dioxan, enthält.

14. Verwendung von heterobifunktionellen Verbindungen mit wenigstens zwei unterschiedlichen funktionellen Gruppen pro Molekül zur Kopplung von Substanzen, insbesondere Allergenen, an Festphasen, wobei eine erste Gruppe das heterobifunktionelle Molekül kovalent an die Festphase bindet und eine zweite Gruppe photochemisch aktivierbar ist.

15. Trägersystem für den Nachweis von Allergien mittels *in vitro* Allergiediagnostikverfahren bestehend aus Trägerelementen (10) und an deren Oberfläche gekoppelten Allergenen (16), dadurch **gekenn-zeichnet**, daß die Allergene (16) über heterobifunktionelle Moleküle (14) kovalent an freie Bindungsstellen (12) der Trägeroberfläche gebunden sind.

16. Trägersystem nach Anspruch 15, dadurch **gekennzeichnet**, daß die heterobifunktionellen Moleküle wenigstens zwei funktionelle Gruppen aufweisen.

17. Trägersystem nach Anspruch 16, dadurch **gekennzeichnet**, daß eine der funktionellen Gruppen zur Bindung der Allergene photoaktivierbar ist.

18. Trägersystem nach einem der Ansprüche 15 bis 17, dadurch **gekennzeichnet**, daß das heterobifunktionelle Molekül ein Azidobenzoesäurederivat ist.

19. Trägersystem nach einem der Ansprüche 15 bis 18, dadurch **gekennzeichnet**, daß zwischen dem heterobifunktionellen Molekül (14) und der Trägeroberfläche des Trägers (10) Proteine (11) oder Proteingemische angeordnet sind.

20. Trägersystem nach einem der Ansprüche 15 bis 19, dadurch **gekennzeichnet**, daß die Trägerelemente als Matrix in Form von Scheiben, Plättchen, Gel oder ähnlichen Substraten ausgebildet sind.

21. Trägersystem nach Anspruch 20, dadurch **gekennzeichnet**, daß die Scheiben oder Plättchen aus Cellulose, Nylon o.dgl. bestehen.

22. Trägersystem nach Anspruch 20, dadurch **gekennzeichnet**, daß das Gel aus Agarose, Sephadex o.dgl. besteht.

23. Trägersystem nach einem der Ansprüche 15 bis 22, dadurch **gekennzeichnet**, daß die Trägerelemente in Reaktionsgefäße eingesetzt sind.
